# EUROPEAN PATENT APPLICATION

(11) **EP 4 305 960 A1**
(43) Date of publication of application: **17.01.2024**
(21) Application number: 23184851.6
(22) Date of filing: 11.07.2023
(51) Int. Cl.: A01N 1/02, C12N 5/077, G01N 3/08, G01N 33/44

(54) **ENGINEERED LEATHER, METHODS OF PRODUCTION AND USES THEREOF**

(30) Priority: 12.07.2022 PT 2022118099
(71) Applicant: Maia & Muller - Biotech, Lda, 4200-135 Porto (PT)
(72) Inventor: GONÇALVES MAIA, MARIA JOÃO, 4350-170 PORTO (PT); AYMÉE MULLER, MARGOT MIRIAM, 06300 NICE (FR)
(74) Representative: Patentree

(57) **Abstract**

The present disclosure relates to an engineered cell-based leather using differentiated skin cells, more specifically reptile or non-human dermal fibroblasts. This leather is cruelty-free and the type of skin reconstruction allows for a tanning process that is ecologic.

## Description

### TECHNICAL FIELD

The present disclosure relates to the production of an engineered cell-based leather using differentiated skin cells, more specifically dermal fibroblasts. This leather is cruelty-free and the type of skin reconstruction allows for a tanning process that is ecologic.

### BACKGROUND

Leather is becoming an unwanted material because of the growing amount of people that are concerned about animal well-being and climate change, as leather is, besides of being a transformation of animal skin, also a very polluting material.

The options to leather that exist on the market, are not real leather but new materials that aim to mimic leather aspect. There are two main types of leather substituting materials: plastic-based leather and bio-based leather. Plastic-based leather is not eco-friendly and bio-based leather existing solutions do not have the same properties as real leather.

There are three subtypes of bio-based leather: plant based, microorganism based and cellular based. Only plant-based and microorganism-based alternatives are currently on the market.

A recent report revealed that sustainability influences purchase behaviour of 82% of true luxury consumers driven by environmental, animal and ethical manufacturing concerns. 36 % of luxury consumers from the Z generation point animal care as the sustainable criteria they value the most when purchasing luxury goods and 35% point environmental care. Companies that continue to use traditional leather will lose clients consistently over time.

Document US20160348078 A1 described an engineered animal skin, hide, and leather comprising a plurality of layers of collagen formed by cultured animal collagen-producing (e.g., skin) cells. Layers may be formed by elongate multicellular bodies comprising a plurality of cultured animal cells that are adhered and/or cohered to one another; wherein the elongate multicellular bodies are arranged to form a substantially planar layer for use in formation of engineered animal skin, hide, and leather.

Document US10273549 B2 discloses methods for obtaining synthetic leather improved barrier function. The synthetic leather comprises forming an artificial dermal layer comprising a fibroblast; and tanning at least a portion of said artificial dermal layer, thereby forming said synthetic leather, wherein said fibroblast is differentiated from an induced pluripotent stem cell.

Both solutions only described the methods for obtain the skin or hide, but none provide a solution to obtain as stable material suitable to be used as substitute for leather.

Document WO2018/185246 relates to a bioartificial reptile leather. A further aspect of this document relates to an article of clothing or of leather goods comprising at least a portion which incorporates a bioartificial reptile leather. Another aspect relates to the use of isolated keratinocytes, isolated dermal stem cells and mesenchymal stem cells, melanocytes, or immortalized fibroblast cells, obtained from a hatched reptile egg for in vitro production of a bioartificial reptile leather product.

Document WO2018/185246 also relates to a method for isolating cells from the chorioallantois from a hatched reptile egg for use in production of bioartificial skin and leather.

These facts are disclosed in order to illustrate the technical problem addressed by the present disclosure.

### GENERAL DESCRIPTION

Leather is a unique material mostly due to its mechanical characteristics. Indeed, bio or plastic based leather products that some companies propose do not compare to the original leather regarding their properties. The engineered leather of the present disclosure eliminates the concerns about animal cruelty and ecology and allows companies to continue to profit of leather characteristics in their products with no risk of losing clients.

In an embodiment, the use of stem cells to prepare cell-based leather allows the implementation of suspension cell cultures on scale-up processes. However, the use of adherent cells, such as fibroblasts, requires a completely different setup to allow the scale up of these cells in an adherent fashion bioreactor of continuous flow.

The present disclosure relates to an engineered cell-based leather comprising adult dermal fibroblasts. In an embodiment, the scale-up of adherent fibroblasts cells can be made recuring to microcarriers where cells are attached to and proliferate upon these carriers that are themselves in suspension. The described method allows the preparation of a stable material that can be used as a leather substitute.

An aspect of the present disclosure relates to the production of an engineered cell-based leather from differentiated skin cells, such as dermal fibroblasts. In an embodiment, the obtained cell-based leather is cruelty-free and is compatible with tanning processes, including ecologic tanning processes; preferably the tanning process comprises the use of vegetable tanning agents like genipin, an aglycone extracted from gardenia flowers; pyrogallol tannins extracted from oak or chestnut; or catechol tannins extracted from mimosa or willow.

The present disclosure relates to an engineered cell-based leather comprising at least a layer comprising dermal fibroblasts; wherein the dermal fibroblasts are selected from reptile dermal fibroblasts or non-human dermal fibroblasts, 0.5 to 50 % (w/w) of chondroitin sulphate; and 5 to 80 % (w/w) of collagen, wherein the weight ratio between collagen and chondroitin sulphate (collagen/chondroitin sulphate) ranges from 1:1 to 10:1; and wherein the maximum tensile strength of the engineered cell-based leather is at least 50 kPa, and/or the Young's modulus of the engineered cell-based leather is at least 5 kPa, measured by a uniaxial tensile test with a maximum force of 1kN.

Young's modulus is a measure of the stiffness of an elastic material, and it is defined as the ratio of stress to strain. This parameter measures the resistance of a material to elastic deformation under load. Thus, a stiff material typically has high Young's modulus values, while a flexible material has a low Young's modulus and changes its shape considerably. Tensile strength is a measure of resistance of a material and is defined as the amount of load or stress that a material can handle until it stretches and breaks.

In an embodiment, the engineered cell-based leather is tanned, preferably with an aqueous solution comprising a crosslinker. In a further embodiment, the crosslinker is a crosslinker that reacts with lysine, acting through a cross-linking mechanism involving the amino groups of lysine and other amino acids through the formation of pyridine intermediaries, like glutaraldehyde. In a yet further embodiment, the crosslinker is selected from glutaraldehyde, genipin, pyrogallol tannins, catechol tannins, or mixtures thereof. Preferably the crosslinker is genipin.

In an embodiment, the tensile strength of the engineered cell-based leather ranges from 50 kPa to 50 MPa. In a preferred another embodiment, the tensile strength of the engineered cell-based leather ranges from 1 MPa to 30 MPa.

In an embodiment, the Young's modulus of the engineered cell-based leather ranges from 5 kPa to 20 MPa. In a preferred embodiment, the Young's modulus of the engineered cell-based leather ranges from 1 MPa to 10 MPa.

In the state of the art, the mechanical properties may be measured by different methods. In an embodiment, the mechanical properties can be measured following the standard ISO 3376:2020. In another embodiment, the mechanical properties, namely tensile strength and Young's modulus, can be measured by uniaxial tensile tests performed using a tensile machine, wherein engineered leather samples are stretched at suitable rate, preferably a rate ranging from 5mm/min to 10mm/min.

In an embodiment, the chondroitin sulfate is chondroitin-6-sulfate.

In an embodiment, the weight ratio collagen/chondroitin sulfate may range from 1:1 to 6:1, preferably from 2:1 to 6:1.

In an embodiment, the ratio between the mass of collagen and the number of dermal fibroblast ranges from 3mg/1 ×10⁶ cells to 10mg/1 ×10⁶ cells.

In an embodiment, the ratio between the mass of collagen and the number of dermal fibroblasts may range from 4mg/1 ×10⁶ cells to 6.7mg/1×10⁶ cells.

In an embodiment, the ratio between the mass of chondroitin sulfate and the number of dermal fibroblasts (mg of chondroitin sulphate/number of cells), per milligram of collagen, ranges from 0.25mg/1×10⁵ cells to 1mg/3.5×10⁵ cells.

In an embodiment, the non-human mammal differentiated skin cells (i.e., dermal fibroblasts) are isolated from bovine, porcine, equine, ovine, or caprine animals, among others.

In an embodiment, the reptile skin cells are isolated from snake, crocodile, lizard, iguana, gecko, or serpent, among others.

In an embodiment, the collagen is animal collagen, plant-based collagen or bacterial collagen.

Another aspect relates to a method of production of the engineered cell-based leather of the present disclosure, the method comprising the following steps:
culturing adherent dermal fibroblast cells for at least 7 days, at 30°C to 37°C, and 5% CO₂;
harvesting the dermal fibroblast cells and mixing the harvested dermal fibroblast cells with a collagen/chondroitin sulphate mixture;
pouring the mixture into a container and culture the cells for 4 to 12 days to obtain a cell-based layer;
soaking and pickling the obtained cell-based layer;
tanning the cell-based layer;
stretching and drying the cell-based layer to obtain the engineered cell-based leather.

In an embodiment, the mass ratio between collagen and chondroitin sulphate ranges from 1:1 to 6:1, preferably 2:1 to 6:1.

In an embodiment, the chondroitin sulfate is chondroitin-6-sulfate.

In an embodiment, collagen is type I collagen. In a further embodiment, collagen is animal recombinant collagen expressed via non-animal living organisms.

In an embodiment, the soaking step comprises immersing the cell-based layer in an aqueous solution of pH ranging from 8 to 10 and a density of 3 Bé for 10 to 24 hours.

In an embodiment the pickling step comprises immersing the cell-based layer in a solution of pH ranging from 2 to 4 comprising 1 % (v/v) sulfuric acid and a density of 9° Bé.

In an embodiment, the tanning step comprises immersing the cell-based layer in a solution comprising 1 to 5% (v/v) of a crosslinker. In a further embodiment, the crosslinker is selected from glutaraldehyde, genipin, pyrogallol tannins, catechol tannins, or mixtures thereof.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of the method for obtaining an eco- friendly and cruelty-free engineered cell-based leather.
**Figure 2****:** Embodiment of the results of the stiffness (represented by the Young's modulus, measured by a uniaxial tensile test with a maximum force of 1kN) of reconstructed skin samples. (-) samples reconstructed in the absence of chondroitin-6-sulfate; (+) samples reconstructed in the presence of chondroitin-6-sulfate (collagen:chondroitin sulfate mass ratio of 1:0.25). ^{∗∗∗} p<0.001 (T.test).
**Figure 3****:** Embodiment of the results of the tensile strength (Resistance) of reconstructed skin samples (measured by a uniaxial tensile test with a maximum force of 1kN). (-) samples reconstructed in the absence of chondroitin-6-sulfate; (+) samples reconstructed in the presence of chondroitin-6-sulfate (collagen:chondroitin sulfate mass ratio of 1:0.25). ^{∗∗∗} p<0.001 (T.test).
**Figure 4****:** Embodiment of the cell-based layer obtained after 1 day of culture of dermal fibroblasts embedded in a collagen/chondroitin sulphate mixture, as described in the present disclosure.
**Figure 5****:** Embodiment of the cell-based layer obtained after 5 days of culture of dermal fibroblasts embedded in a collagen/chondroitin sulphate mixture, as described in the present disclosure.
**Figure 6****:** Embodiment of the engineered cell-based leather after the tanning process with glutaraldehyde.
**Figure 7****:** Embodiment of results from the Scanning Electron Microscopy (SEM) analysis to a disclosed engineered cell-based leather after tanning process with glutaraldehyde. Scale bar: 2µm.

### DETAILED DESCRIPTION

The present disclosure relates to the production of engineered cell-based leather using differentiated skin cells, more specifically dermal fibroblasts. This leather is cruelty-free and the type of skin reconstruction allows for a tanning process that is ecologic. Besides the compatibility with ecologic tanning processes, the preparation of the described engineered cell-based leather saves up to 60% of water, as compared to a typical tanning process, and also reduces de CO₂ amount release to the atmosphere since there is no need of animal farming, as occurs for traditional leather preparation processes.

In an embodiment, cells are isolated from a skin sample, preferably with a size smaller than 1 cm², of non-human mammal or reptile. In a preferred embodiment, the skin sample is collected post-mortem. Then, the sample is soaked in 70% (v/v) ethanol for 1 minute and minced with a scalpel in a suitable cell culture media at 30°C; after, the minced explant is transferred to a cell culture dish and incubated at a suitable temperature, preferably ranging from 30°C to 37°C, 5% CO₂. At last, cells were harvested at or near cell confluence.

In an embodiment of dermal reconstruction, the isolated fibroblasts were incubated for 7 days at 37°C and 5% CO₂ in RPMI supplemented medium, changing the medium each second day.

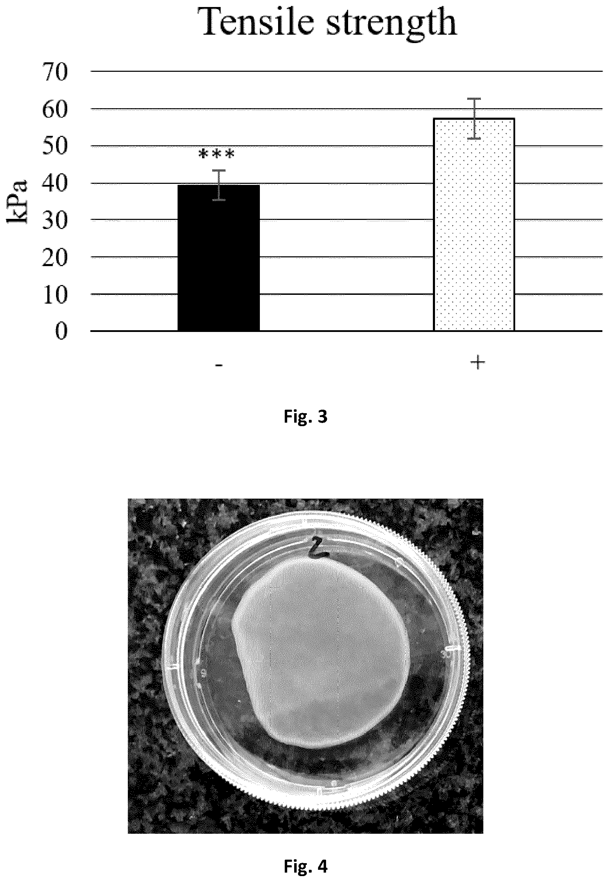

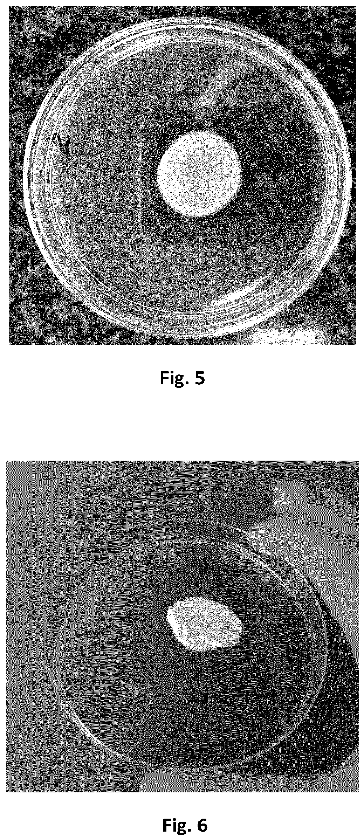

In an embodiment of dermal reconstruction, the isolated fibroblasts were incubated in a stirred tank, attached to microcarriers, at 37°C and 5% CO₂ in RPMI supplemented medium. When at confluence, the fibroblasts were harvested and moved to be cultured on a collagen/chondroitin sulphate tri-dimensional matrix for 4 to 12 days, preferably a matrix with a ratio of collagen:chondroitin sulfate:fibroblasts (mg:mg:cell number) ranging from 1:0.25:1×10⁵ to 1:1:5×10⁵, preferably 1:0.25:5×10⁵. During these 4 to 12 days, preferably 4 days, fibroblasts rearrange their fibres and contract the matrix, as showed in Figure 4 (cell-based layer after 1 day of culture) and Figure 5 (cell-based layer after 5 days of culture).

In a further embodiment, selected cell culture media were used on the dermal reconstruction process, as follows:
- Medium 1:
   16.74% (v/v) MEM 10X (Gibco, USA, 21430-020)
   5.08% (v/v) NaHCO3 (Gibco, USA. 25080-060)
   0.84% (v/v) L-Glutamine (1,76 mM) (Gibco, USA, 25030-081, 200 mM)
   0.84% (v/v) Sodium Pyruvate (0,88 mM) (Gibco, USA, 11360-039, 100 mM)
   0.84% (v/v) non-essential aminoacid (NEAA) solution (0.88X) (Gibco, USA, 11140-035, 100X)
   0.04% (v/v) Antibiotic-Antimycotic (1X) (Gibco, USA, 15240-096, 100X)
   75.63% (v/v) ultrapure Water (Thermo Fisher Scientific, USA, AM9930)
- Medium 2:
   36.50% (v/v) MEM 25 mM Hepes (Gibco, USA. 32360-026)
   0.36% (v/v) L-Glutamine (2 mM) (Gibco, USA, 25030-081, 200 mM)
   0.36% (v/v) Sodium Pyruvate (1 mM) (Gibco, USA, 11360-039, 100 mM)
   0.36% (v/v) NEAA solution (1X) (Gibco, USA, 11140-035, 100X)
   0.36% (v/v) Antibiotic-Antimycotic (1X) (Gibco, USA, 15240-096, 100X)
   3.65% (v/v) Panexin CD (PanBiotech, Germany, P04-930500)
   NaOH 0.1N (29.20% (v/v)):
   2.92% (v/v) NaOH 1N (Sigma, USA,S2770, 1,0N) + 26.28% (v/v) ultrapure water (Thermo Fisher Scientific, USA, AM9930)
- Solution 1:
   76.59% (v/v) Medium 1
   14.32% (v/v) Panexin CD (PanBiotech, Germany, P04-930500)
   4.55% (v/v) NaOH 0,1N (Sigma, USA, Réf. S2770, 1,0N)
   4.55% (v/v) Medium 2
   5 mg/mL of chondroitin sulfate, preferably chondroitin-6-sulfate (Millipore, 230699)

In an embodiment, the harvested fibroblasts were centrifuged and the resulting pellet resuspended in Medium 2 at 2×10⁶/mL. Then the cellular suspension was gently mixed with a volume of solution 1, followed by the addition of a solution of type I collagen in 0.01M HCl; in a preferred embodiment, the volume ratio between solution 1, cell suspension, and collagen ranged from 2:0.5:4 to 0.5:0.5:5.5. The resulting solution formed a three-dimensional matrix with embedded cells, and was then poured in a container suitable for cell culture, such as a petri dish, and cultured for 12 days, with change of media each five days, in an incubator at 5% CO₂, with a temperature ranging from 32 to 37 °C.

In an embodiment of leather tanning, the cell-based layer was weighted and treated overnight with a 200% w/v soaking solution comprising water, supplemented with sodium carbonate to adjust the pH of the solution to pH 9.5, and with sodium chloride to adjust the density to 3 Bé (Baumé degrees). One day 2, i.e., after the overnight soaking treatment, the dermis was first treated with a pickling solution (400%w/v) pH 3 for 2h followed by a treatment with a tanning solution (400%w/v) pH 3 for 5h. The pickling solution comprised water, 1% (v/v) sulfuric acid and a density of 9° B6 (by addition of sodium chloride). The tanning solution comprised 1.5% (v/v) of glutaraldehyde and a density of 9° B6 (by addition of sodium chloride). After the 5h of the tanning treatment, the pH was adjusted to 6.5 and the treatment continued for more 1h30. The resulting tanned dermis was then rinsed in 400% (w/v) water overnight. On day 3, the dermis was stretched transferred to a dry surface, and dried by an air-drying process.

For the scope and interpretation of the present disclosure, the Baumé degree of the different solutions was measured by a hydrometer calibrated at 20°C.

In an embodiment, as comparative example, fibroblasts were also cultured in the absence of chondroitin-6-sulfate.

In an embodiment, mechanical tests were performed at different stages of dermal reconstruction (cell-based layer formation) and tanning to evaluate the mechanical properties of the obtained engineered cell-based skin. Uniaxial tensile tests were performed using a Instron 34SC tensile machine. Samples (10mmx20mm) were stretched at 5mm/min and maximum force of 1kN. Data were analysed to obtain the tensile strength and Young Modulus.

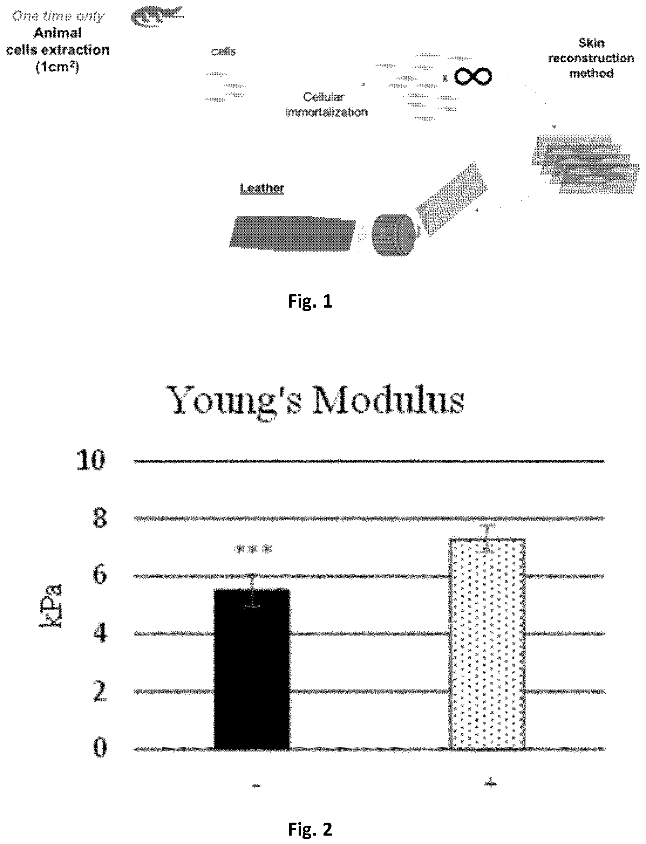

Figure 2 shows an embodiment of the stiffness of the reconstructed dermal layer, represented by the average of measured Young's Modulus, of 10 samples of reconstructed skin of each condition, i.e., cultured in presence and absence of chondroitin sulphate. Samples were cut in small strips (10mm × 20mm) and uniaxial test was performed using a tensile machine (Instron 34SC). It is possible to observe a significant higher Young's modulus for cells cultured in the presence of chondroitin-6-sulfate (+), showing that the presence of this sulphated glycosaminoglycan in culture media improves the stiffness of the resulting reconstructed dermal layer.

Figure 3 shows the resistance of the reconstructed dermal layer, represented by the average of stress at maximum load (tensile strength), of 10 samples of reconstructed skin of each condition, i.e., cultured in presence and absence of chondroitin sulphate. Samples were cut in small strips (10mm × 20mm) and uniaxial test was performed using a tensile machine (Instron 34SC). Similarly, the presence of chondroitin sulfate on the culture media increased the stress at maximum load, meaning that the resulting reconstructed dermal layer is more resistant to tear than the dermal layer obtained in the absence of chondroitin sulfate.

In an embodiment, the amount of collagen in the engineered cell-based leather ranged from 5 to 80% (w/w). Different methods are available to quantify collagen present in biological samples such as hydroxyproline quantification assay. As example, the Millipore MAK008 kit can be used for collagen quantification. According to the manufacturer, 6 M HCl are added to 2.0-mL, screw-top, polypropylene tubes as 20 µL per 1 mg tissue, and these samples are hydrolyzed for 24 hours under a tight lid at 110°C. Next, the tubes are mixed and centrifuged at 10,000 *g* for 3 minutes. Afterwards, 10 µL supernatant are diluted with 190 µL water in new 1.5-mL tubes. Then, 20 µL are transferred to 96-well plates. The 96-well plates were placed in a 60°C oven to dry samples. Then 100 µL Chloramine T/Oxidation buffer mixture (part of the kit) was added to each reaction well and standard well. The plates were incubated at 25°C for 10 minutes. Then 100 µL Diluted DMAB Reagent (MilliporeSigma) was added to each reaction well and standard well, and then the plates were incubated in a 60°C oven for 90 minutes. The absorbance of each well was measured at 560 nm with a spectrophotometer.

In an embodiment, the amount of chondroitin sulfate collagen in the engineered cell-based leather ranged from 0.5 to 50 % (w/w). Different methods are available to quantify chondroitin sulfate present in biological samples such as High-Performance Liquid Chromatography (HPLC). In an embodiment, tissues were minced and then homogenized in 4 vol. of acetone with ice cooling. After centrifuging at 1000g for 15 min, the precipitate was washed with ether and dried in vacuum. The defatted dry sample was suspended in 1.0 M NaOH and kept overnight at 4°C. After neutralizing with 1 M HCl, the solution was boiled for 2 min. The solution was then mixed with 10 mg/ml collagenase in 50 mM Tris- acetate buffer (pH 8.0), and the solution was stood at 37°C for 3 h. Afterwards, the solution was mixed with 10 mg/ml actinase E solution in 0.01 M Tris-acetate buffer (pH 8.0), and the reaction mixture was heated at 45°C for 24 h. Then 50 mM acetic acid containing 15% NaCl was added, and the solution was boiled for 5 min. After centrifuging at 2300g for 15 min, the supernatant was neutralized with 0.25 M NaOH. To the solution, cold ethanol was added, and the solution stand overnight at 4°C. After centrifuging at 2300g for 15 min, the precipitate was washed with 80% ethanol and acetone, and dried in vacuum. Then, a 20-ml portion of the sample solution was mixed with 10 ml of 0.1 M Tris-acetic acid (pH 9.1) and 10 ml of chondroitinase ABC solution (1 unit/400 ml), and then the solution mixture was held at 37°C for 1 h. Furthermore, the reaction mixture was mixed with 20 ml of 0.5 M acetate buffer containing 40 mM zinc acetate (pH 5.4) and 20 ml of Streptomyces hyaluronidase (100 TRU/ml) in series, and the solution mixture was held at 50°C for 2 h. A 5-ml portion of the reaction mixture was injected into the HPLC for analysis.

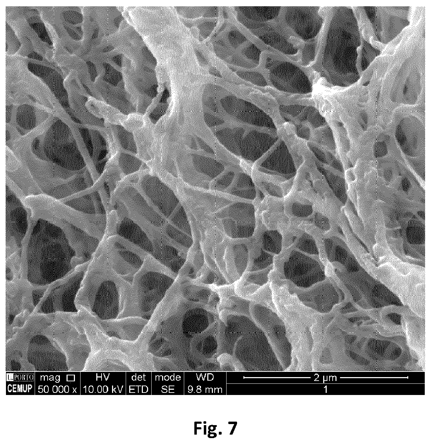

Figure 7 shows an embodiment of the results of SEM of the engineered cell-based leather after tanning process with glutaraldehyde. A strip (10mm × 10mm) of the sample was mounted, dried in low-vacuum, sputter-coated with gold, and examined using a FEI QUANTA 400FEG ESEM/EDAX Genesis X4M at 15 kV.

The term "comprising" whenever used in this document is intended to indicate the presence of stated features, integers, steps, components, but not to preclude the presence or addition of one or more other features, integers, steps, components or groups thereof.

Where singular forms of elements or features are used in the specification of the claims, the plural form is also included, and vice versa, if not specifically excluded. For example, the term "a cell" or "the cell" also includes the plural forms "cells" or "the cells," and vice versa. In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process.

Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

## Claims

1. An engineered cell-based leather comprising at least a layer comprising:
dermal fibroblasts, wherein the dermal fibroblasts are selected from reptile dermal fibroblasts or non-human mammal dermal fibroblasts;
0.5 to 50 % (w/w) of chondroitin sulphate;
and 5 to 80 % (w/w) of collagen,
wherein the weight ratio between collagen and chondroitin sulphate ranges from 1:1 to 10:1; and
wherein the tensile strength of the engineered cell-based leather is at least 50 kPa; and/or the Young's modulus of the engineered cell-based leather is at least 5 kPa, measured by a uniaxial tensile test with a maximum force of 1kN.

2. The engineered cell-based leather according to the previous claim wherein the engineered cell-based leather is tanned, preferably with an aqueous solution comprising a crosslinker, preferably glutaraldehyde, genipin, pyrogallol tannins, catechol tannins, or mixtures thereof.

3. The engineered cell-based leather according to the previous claim wherein the crosslinker is a crosslinker that reacts with lysine.

4. The engineered cell-based leather according to any of the previous claims wherein the tensile strength of the engineered cell-based leather ranges from 50 kPa to 50 MPa, preferably from 1 MPa to 30 MPa.

5. The engineered cell-based leather according to any of the previous claims wherein the Young's modulus of the engineered cell-based leather ranges from 5 kPa to 20 MPa, preferably from 1 MPa to 10 MPa.

6. The engineered cell-based leather according to any of the previous claims wherein the chondroitin sulfate is chondroitin-6-sulfate.

7. The engineered cell-based leather according to any of the previous claims wherein the weight ratio between collagen and chondroitin sulfate ranges from 1:1 to 6:1, preferably from 2:1 to 6:1.

8. The engineered cell-based leather according to any of the previous claims wherein the ratio between the mass of collagen and the number of dermal fibroblasts ranges from 3mg/1×10⁶ cells to 10mg/1×10⁶ cells, preferably from 4mg/1×10⁶ dermal fibroblasts to 6.7mg/1×10⁶ dermal fibroblasts.

9. The engineered cell-based leather according to any of the previous claims wherein the ratio between the mass of chondroitin sulfate and the number of dermal fibroblasts per milligram of collagen ranges from 0.25mg:1×10⁵ cells to 1mg:3.5×10⁵ cells.

10. The engineered cell-based leather according to any of the previous claims wherein the non-human mammal dermal fibroblasts are isolated from bovine, porcine, equine, ovine, or caprine animals.

11. The engineered cell-based leather according to any of the previous claims wherein the reptile skin cells are isolated from snake, crocodile, lizard, iguana, gecko, or serpent.

12. The engineered cell-based leather according to any of the previous claims wherein the collagen is animal collagen, plant-based collagen or bacterial collagen.

13. A method of production of an engineered cell-based leather as described in any of the previous claims the method comprising the following steps:
culturing adherent dermal fibroblast cells for at least 7 days at 30°C to 37°C and 5% CO₂;
harvesting the dermal fibroblast cells and mixing the harvested dermal fibroblast cells with a collagen/chondroitin sulphate mixture, preferably wherein the mass ratio between collagen and chondroitin sulphate ranges from 1:1 to 6:1, preferably 2:1 to 6:1;
pouring the mixture into a container and culture the cells for 4 to 12 days to obtain a cell-based layer;
soaking and pickling the obtained cell-based layer, wherein the soaking step comprises immersing the engineered cell-based leather in an aqueous solution of pH ranging from 8 to 10 and a density of 3 Bé for 10 to 24 hours and wherein the pickling step comprises immersing the engineered cell-based leather in a solution of pH ranging from 2 to 4 comprising 1 % (v/v) sulfuric acid and a density of 9° Bé; tanning the cell-based layer;
stretching and drying the cell-based layer to obtain the engineered cell-based leather.

14. The method according to any the previous claim wherein the chondroitin sulfate is chondroitin-6-sulfate and/or the collagen is type I collagen.

15. The method according to any of the previous claims 16-21 wherein the tanning step comprises immersing the engineered cell-based leather in a solution comprising 1 to 5% (v/v) of a crosslinker, preferably wherein the crosslinker is selected from glutaraldehyde, genipin, pyrogallol tannins, catechol tannins, or mixtures thereof.
